# FASCICULE DE BREVET EUROPEEN

(11) **EP 0 641 808 B1**
(45) Date de publication et mention de la délivrance du brevet: **26.02.1997**
(21) Numéro de dépôt: 94401961.1
(22) Date de dépôt: 02.09.1994
(51) Int. Cl.: C08F 20/36, G02F 1/35, C07C 245/08

(54) **Matériau polymère réticulable, utilisable en optique non linéaire, et son procédé d'obtention**
Vernetzbares Polymer verwendbar in nicht linearen optischen Vorrichtungen und Verfahren zu seiner Herstellung
Curable polymer, for use in non-linear optical devices and process for its preparation

(30) Priorité: 06.09.1993 FR 9310572
(43) Date de publication de la demande: 08.03.1995
(73) Titulaire: FRANCE TELECOM, 75015 Paris (FR)
(72) Inventeur: Foll, Franck, Résidence Le Laurencin (B), F-34090 Montpellier (FR); Bosc, Dominique, F-22300 Lannion (FR); Liang, Julienne, F-94260 Fresnes (FR); Rousseau, Alain, F-34090 Montpellier (FR); Boutevin, Bernard, F-34000 Montpellier (FR)
(74) Mandataire: Burbaud, Eric

(56) Documents cités:
- FR-A- 2 630 744
- GB-A- 2 246 138

## Description

La présente invention concerne les matériaux actifs en optique non linéaire, qui sont utilisés notamment dans le domaine des télécommunications optiques et du traitement optique des signaux. Par exemple, ces matériaux peuvent être utilisés pour la génération d'harmoniques, la transposition de fréquence, les mémoires optiques, les modulateurs optiques, les amplificateurs optiques, etc...

Parmi les différents matériaux utilisables en optique non linéaire, les matériaux organiques polymérisés présentent des performances compétitives par rapport aux matériaux inorganiques tels que l'arséniure de gallium et le niobate de lithium.

En effet, les matériaux organiques permettent de fabriquer des circuits optiques intégrés multicouches par des techniques déjà éprouvées sur les circuits intégrés électroniques (notamment photolithographie), contrairement aux matériaux minéraux qui doivent être utilisés sous forme de monocristaux. Il en résulte un moindre coût de fabrication des composants optiques.

De plus, l'hybridation des matériaux organiques est plus facile que dans le cas des matériaux minéraux.

En outre, les matériaux organiques permettent des traitements du signal beaucoup plus rapides que les matériaux minéraux, du fait de leur vitesse de changement d'état beaucoup plus grande.

Enfin, dans le cas où ces matériaux sont utilisés dans un modulateur optique, les matériaux organiques permettent de réduire la tension de commande et la longueur d'interaction entre l'onde lumineuse et le champ électrique de commande, ce qui permet une utilisation plus facile et un plus grand débit d'informations.

Les polymères utilisables en optique non linéaire sont généralement utilisés sous forme de film. Ils comportent un squelette carboné sur lequel sont rattachés des groupements latéraux optiquement non linéaires (GONL) ou chromophores. Pour que le matériau soit actif en optique non linéaire, les GONL doivent être orientés, de façon à rendre le milieu non centro-symétrique.

Cette orientation se fait au moyen d'un champ électrique de polarisation transversal au film de polymère, sous une tension en général de l'ordre de 100 V/µm d'épaisseur de film ou supérieure, pendant que le matériau est chauffé à une température voisine de sa température de transition vitreuse ou légèrement supérieure.

Lorsque le matériau est refroidi à une température inférieure à sa température de transition vitreuse, les GONL conservent leur orientation imposée par le champ électrique. Toutefois, les GONL perdent peu à peu cette orientation au fil du temps, et ce d'autant plus rapidement que leur température de transition vitreuse est basse. Comme la température de transition vitreuse n'est jamais très élevée, le matériau polymère a donc des propriétés optiques non linéaires qui sont instables au cours du temps.

En outre, les composants optiques voient généralement leur température s'élever pendant leur utilisation, ce qui accélère encore le processus de relaxation de l'orientation des GONL.

Pour remédier à cette instabilité, on a tenté de bloquer les GONL après leur orientation, par des liaisons chimiques.

Ainsi, on s'est efforcé de bloquer les GONL en les faisant participer au processus de polymérisation, en maintenant un champ électrique d'orientation pendant la polymérisation. Toutefois, il est particulièrement difficile de contrôler en même temps la polymérisation et l'orientation des GONL sous l'effet du champ électrique, au voisinage de la température de transition vitreuse. En outre, les concentrations en GONL que l'on peut ainsi obtenir sont relativement faibles.

Par ailleurs, le document FR-A- 2 630 744 divulgue un matériau polymère utilisable en optique non linéaire, dans lequel les GONL sont maintenus orientés par une réticulation du polymère qui est réalisée pendant l'application d'un champ électrique de polarisation des GONL, après polymérisation. Toutefois, la réticulation est obtenue par des liaisons entre les squelettes carbonés des chaînes polymérisées, et non par des liaisons chimiques concernant directement les GONL. Par conséquent, la mobilité des GONL, si elle est gênée par la réticulation, n'est pas pour autant supprimée.

B.K. MANDAL et al (Makromol. Chem. Rapid Commun., 12, 63-68 (1991)) ont proposé de doper un polymère possédant des groupements photoréticulables, avec un colorant actif en optique non linéaire, qui formait les GONL du polymère et qui possédait ces mêmes groupements photoréticulables à ses deux extrémités. La réticulation permettait ainsi de bloquer les GONL eux-mêmes par réticulation après polarisation, mais au dire même des auteurs, ce système devait être optimisé pour être exploitable. En particulier, les concentrations en GONL que l'on pouvait ainsi obtenir étaient faibles.

S. MULLER (thèse de l'Université Paris VI, du 1er juillet 1992), a divulgué un polymère utilisable en optique non linéaire, dans lequel les GONL pouvaient se lier entre eux par chauffage pendant leur polarisation par un champ électrique. Toutefois, les coefficients électro-optiques obtenus étaient trop faibles. En effet, selon une hypothèse vraisemblable, la réaction d'ancrage des GONL intervenait avant que leur orientation effective ait eu lieu.

Cécil V. FRANCIS et al. (Chem. Mater. 1993, 5, 506-510) ont divulgué un matériau utilisable en optique non linéaire, obtenu par réaction entre du tolonate HDT (un triisocyanate) et un chromophore qui possédait un groupement hydroxyle à une extrémité, et une fonction amine à son extrémité opposée. Les chromophores ont été mélangés au tolonate HDT et chauffés à 50°C, ce qui a provoqué la réaction de la fonction amine avec certains groupements isocyanates du tolonate HDT, en donnant des oligomères pouvant former un film. Puis, le film a été chauffé à 75°C, pendant l'application d'un champ électrique de polarisation des chromophores, ce qui a provoqué la réticulation du matériau du film, par réaction entre les groupements hydroxyle des chromophores et les groupements isocyanate restants du tolonate HDT. Le matériau obtenu présente toutefois un coefficient électro-optique médiocre.

La présente invention a surtout pour but de stabiliser les GONL après leur orientation, pour obtenir un polymère utilisable en optique non linéaire qui présente à la fois de bonnes propriétés optiques non linéaires et une grande stabilité à température élevée, avec une concentration en GONL suffisante .

A cet effet, l'invention a pour objet un matériau réticulable actif en optique non linéaire, comportant un polymère sur lequel sont greffés latéralement des chromophores actifs en optique non linéaire et orientables sous l'effet d'un champ électrique de polarisation, lesdits chromophores comportant un groupe fonctionnel d'ancrage pour stabiliser l'orientation des chromophores par réticulation après leur polarisation par un champ électrique, caractérisé en ce que les chromophores correspondent à la formule (I) suivante : 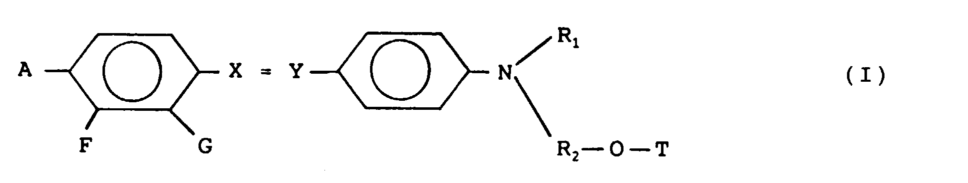 dans laquelle :
- T est le polymère,
- A est un groupement -NO₂, -CN, ou -C(CN)=C(CN)₂,
- R1 est un atome d'hydrogène, un groupement méthyle ou éthyle,
- R2 est un groupement CH₂, C₂H₄, C₃H₆ ou C₄H₈,
- et :
- soit X = Y est un groupement azoïque (X et Y sont des atomes d'azote), F étant un atome d'hydrogène et G étant un groupement -C(O)-O-W ou un groupement -C(O)-O-(CH₂)ₙ-O-W, n étant un entier compris entre 2 et 6 et W étant un atome d'hydrogène ou un groupement dimérisable, G constituant alors le groupe fonctionnel d'ancrage susmentionné,
- soit X = Y est un groupement azométhine (X est un groupement CH et Y est un atome d'azote, ou X est un atome d'azote et Y est un groupement CH), F étant un groupement -O-W ou -R₃-O-W, W étant un atome d'hydrogène ou un groupement dimérisable, R₃ étant un groupement -O-C(O)-R₄- ou -O-R₄-, où R₄ est un alkyle C₁ à C₆, et G étant un atome d'hydrogène, F constituant alors ledit groupe fonctionnel d'ancrage,
- soit X = Y est un groupement azométhine (X est un groupement CH et Y est un atome d'azote, ou X est un atome d'azote et Y est un groupement CH), G étant un groupement -O-W ou -R₃-O-W, W étant un atome d'hydrogène ou un groupement dimérisable, R₃ étant un groupement -O-C(O)-R₄- ou -O-R₄-, où R₄ est un alkyle C₁ à C₆, et F étant un atome d'hydrogène, G constituant alors ledit groupe fonctionnel d'ancrage,
ledit matériau comportant en outre au moins un groupe fonctionnel d'ancrage complémentaire, susceptible de se lier au groupe fonctionnel d'ancrage porté par le chromophore lors de la réticulation.

Les chromophores sont orientés au moyen d'un champ électrique de polarisation, puis on les bloque dans leur position orientée par réticulation thermochimique ou photochimique, par liaison du groupe fonctionnel d'ancrage des chromophores avec le groupe fonctionnel réticulable complémentaire.

Le matériau polymère réticulé obtenu présente un excellent coefficient électro-optique, et ses caractéristiques optiques présentent une grande stabilité à température élevée.

L'invention a aussi pour objet ce matériau polymère réticulé.

Selon une forme de réalisation avantageuse de l'invention, lorsque X = Y est un groupement azométhine et que F est un groupement -R₃-O-W, R₃ est un groupement -O-R₄-.

Selon une autre forme de réalisation avantageuse de l'invention, lorsque X = Y est un groupement azométhine et que G est un groupement -R₃-O-W, R₃ est un groupement -O-C(O)-R₄-.

Le motif du polymère qui comprend le chromophore peut être choisi dans la famille des acrylates ou des méthacrylates ou dans la famille des styréniques, dans la famille des α-haloacrylates, dans la famille des cétones, ou dans la famille des éthers vinyliques.

En particulier, le motif du polymère qui comprend le chromophore peut avoir l'une des formules suivantes : 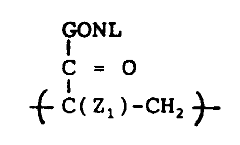 et 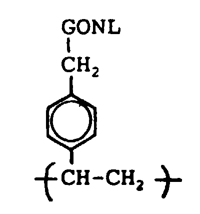 Z₁ étant un atome d'hydrogène, de fluor, ou de chlore, ou un groupement méthyle et GONL représentant le chromophore.

Le polymère peut comporter, outre le motif qui comprend le chromophore, au moins un motif additionnel, le motif qui comprend le chromophore représentant une proportion molaire comprise entre 10 et 50 % des motifs du polymère.

Ces motifs additionnels peuvent être choisis dans la famille des acrylates ou des méthacrylates, dans la famille des styréniques, dans la famille des esters vinyliques, dans la famille des éthers vinyliques ou dans la famille des carbonates vinyliques.

Ces motifs additionnels peuvent comprendre le groupe fonctionnel d'ancrage complémentaire.

En outre, ces motifs additionnels servent à ajuster les propriétés thermomécaniques du matériau et à ajuster la concentration des chromophores, pour déterminer les propriétés optiques du matériau.

Le groupe fonctionnel d'ancrage complémentaire peut aussi être porté par des molécules additionnelles mélangées intimement au polymère qui porte le chromophore. Ces molécules additionnelles peuvent être soit des molécules polymérisées dont un motif inclut le groupe fonctionnel d'ancrage complémentaire, soit des molécules portant chacune deux groupes fonctionnels d'ancrage complémentaires qui sont de préférence identiques, le radical qui porte les deux groupes fonctionnels d'ancrage complémentaires étant de préférence un radical aryle ou alkyle C₁ à C₁₀.

Selon une première forme de réalisation de l'invention, G est un groupement -COOH et constitue le groupe fonctionnel d'ancrage du chromophore et le groupe fonctionnel d'ancrage complémentaire est un groupement isocyanate, époxyde, hydroxyle, ou -SH.

La réaction de réticulation est alors déclenchée par chauffage, pendant l'application du champ électrique de polarisation.

Dans cette forme de réalisation, le polymère peut comporter avantageusement, outre le motif qui comprend le chromophore, au moins un motif qui présente la formule (III) suivante : 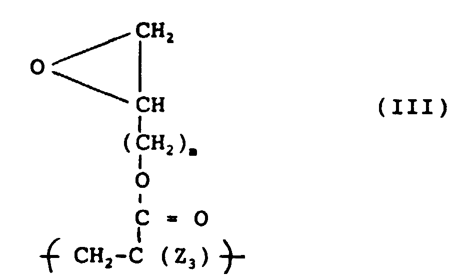 où :
- Z₃ est un atome d'hydrogène, de fluor, ou de chlore ou un groupement méthyle, et
- m est un entier compris entre 1 et 6.

Selon une deuxième forme de réalisation de l'invention, l'un des groupements F et G comporte une fonction alcool et constitue le groupe fonctionnel d'ancrage du chromophore, le groupe fonctionnel d'ancrage complémentaire étant un groupement isocyanate, -COOH, ou chlorure d'acide.

La réaction de réticulation est alors déclenchée par chauffage pendant l'application du champ électrique de polarisation.

Dans cette forme de réalisation, le polymère peut comporter, outre le motif qui inclut le chromophore, au moins un motif qui présente la formule (IV) suivante :

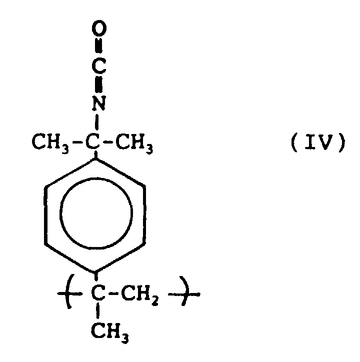

Selon une troisième forme de réalisation de l'invention, W est un groupement dimérisable choisi dans le groupe comprenant :

-C(O)-C(Z₂) = CH₂

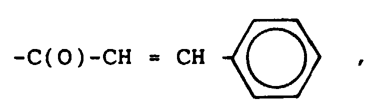

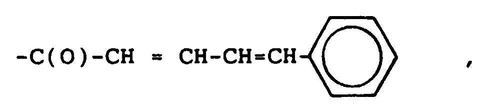

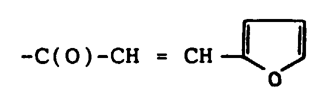

et 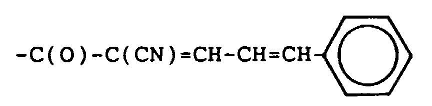 Z₂ étant un atome d'hydrogène, de fluor ou de chlore ou un groupement méthyle, et le groupe fonctionnel d'ancrage complémentaire comportant également un groupement fonctionnel dimérisable choisi dans le même groupe que W.

De préférence, les groupements dimérisables du groupe fonctionnel d'ancrage porté par le chromophore et du groupe fonctionnel d'ancrage complémentaire, sont identiques.

Pour ces différents groupements W, la réaction de dimérisation est déclenchée par insolation, juste après application du champ électrique de polarisation. Toutefois, dans le cas du groupement -C(O)-C(Z₂) = CH₂, la réaction peut aussi être déclenchée par chauffage pendant l'application du champ électrique de polarisation.

Dans cette forme de réalisation, en général, les groupes fonctionnels d'ancrage portés par les chromophores constituent au moins une partie des groupes fonctionnels d'ancrage complémentaires, la réticulation se faisant au moins en partie par dimérisation entre chromophores. La réticulation pourrait même se faire éventuellement uniquement par dimérisation entre chromophores.

Pour préparer le matériau actif en optique non linéaire, une voie préférée consiste à polymériser un monomère de formule (VI) suivante : 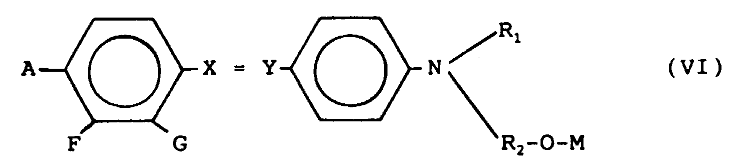 dans laquelle A, F, G, R₁, R₂, sont tels que définis précédemment et M est un groupement choisi dans la famille des radicaux acryliques, méthacryliques et styréniques.

L'invention a aussi pour objet ce monomère.

En particulier, M peut avoir l'une des formules suivantes :
-C(O)-C(Z₁) = CH₂, Z₁ étant un atome d'hydrogène, de fluor, de chlore, ou un groupement méthyle,
et :

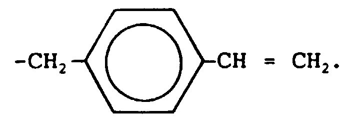

Lorsque X = Y est un groupement azoïque, le monomère de formule (VI) peut être obtenu à partir des composés 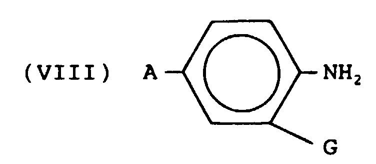 et 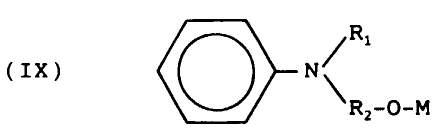 par un procédé comportant d'abord une étape de diazotation du composé (VIII), pour obtenir un sel de diazonium de ce composé, puis une étape de copulation entre ledit sel de diazonium et le composé (IX).

Lorsque M est un groupement de la famille des radicaux acryliques ou méthacryliques (c'est-à-dire en particulier lorsque M correspond à la formule -C(O)-C(Z₁) = CH₂, Z₁ étant tel que défini ci-dessus), le composé (IX) peut être préparé par estérification entre le composé 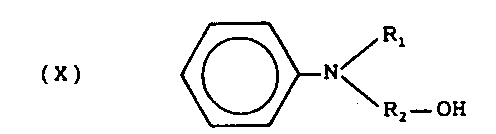 et l'acide M-OH ou son chlorure d'acide.

Lorsque M est un groupement styrénique, le composé (IX) peut être préparé par réaction entre le composé (X) et le chlorométhylstyrène avec catalyse par transfert de phase (CTP).

Lorsque G est un groupement -C(O)-O-(CH₂)ₙ-OH, l'obtention de ce groupement peut comporter une étape d'estérification d'un composé Br-(CH₂)ₙ-OH sur un groupement carboxyle.

Cette estérification peut avoir lieu soit sur le monomère (ce qui est préféré) soit sur le polymère, soit sur le composé (VIII) susmentionné.

Lorsque G est un groupement C(O)-O-W et -O-W est un groupement carboxylique dimérisable, G peut être obtenu par réaction entre un groupement -COOH situé initialement à la place du groupement G sur le chromophore, et un chlorure d'acide Cl-W, ce qui donne l'anhydride G.

Lorsque G est un groupement C(O)-O-(CH₂)ₙ-O-W et -O-W est un groupement carboxylique dimérisable, l'obtention de ce groupement G peut comporter une étape d'estérification entre l'alcool

Br-(CH₂)ₙ-OH

et le chlorure d'acide Cl-W pour obtenir le composé (XI) :

Br-(CH₂)ₙ-O-W,

puis une étape d'estérification entre le composé (XI) et un groupement carboxyle situé initialement à la place du groupement G dans le chromophore.

Le groupement G peut être formé de cette façon soit sur le monomère (ce qui est préféré) soit sur le polymère, soit sur le composé (VIII) susmentionné.

Lorsque X = Y est est un groupement azométhine, l'obtention du monomère peut être obtenue par l'une des trois voies suivantes :
(a) réaction entre 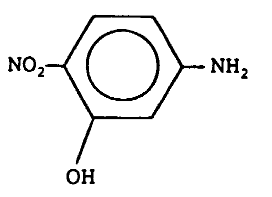 ou 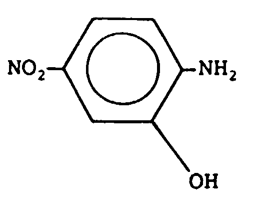 et 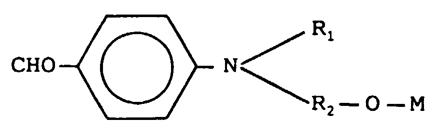
(b) réaction entre 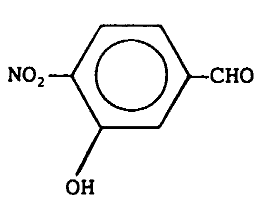 ou 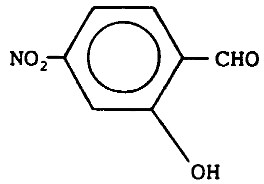 et 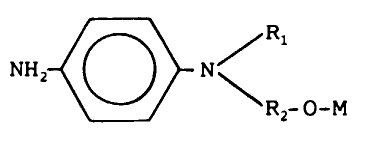
(c) réaction entre 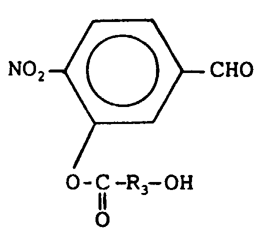 ou 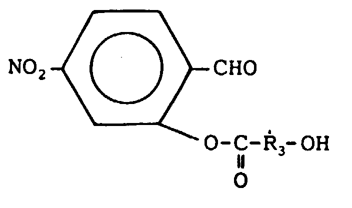 et

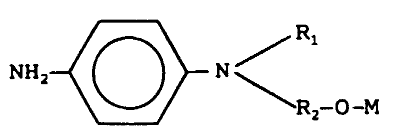

Dans les voies (b) et (c), la diamine peut être obtenue à partir de la paranitroaniline alkylée par réduction de -NO₂ en -NH₂.

Lorsque X = Y est un groupement azométhine et -O-W est un groupement carboxylique dimérisable, le greffage de W peut être effectué par réaction du chlorure d'acide Cl-W sur un groupement hydroxyle du chromophore.

L'invention a aussi pour objet l'utilisation d'un des matériaux selon l'invention dans un composant optique.

Les matériaux selon l'invention sont utilisables dans toutes les applications de l'optique non linéaire, notamment les modulateurs.

De plus, ils peuvent être utilisés en optique linéaire pour réaliser des mémoires mortes optiques, en particulier lorsque la réticulation est photochimique. En effet, après polarisation des chromophores, il est alors possible de réticuler localement le matériau polymère à l'aide d'un rayon lumineux, notamment laser, puis d'éliminer la polarisation dans les zones non réticulées par chauffage de l'ensemble du matériau. On peut ainsi graver des informations sur un support plan, par exemple un disque optique.

Des modes particuliers de réalisation de l'invention sont indiqués dans les revendications dépendantes.

Deux exemples particuliers de réalisation de l'invention, donnés à titre non limitatif, vont maintenant être décrits, en regard du dessin joint, sur lequel :
- la figure 1 représente l'intensité du second harmonique I_{2ω}, d'un matériau selon l'invention, rapportée à son intensité juste après polarisation I_{2ω} max (I_{2ω}/I_{2ω} max en %) en fonction de la température (θ en °C) avec et sans réticulation (respectivement en trait plein et en pointillés), et
- les figures 2 et 3 représentent chacune des spectres d'absorption d'un matériau selon l'invention, respectivement avant polarisation, après polarisation sans réticulation, et après réticulation.

### EXEMPLE 1

### 1°) Synthèse du 2-(N-éthyl-anilino)éthyl méthacrylate

Cette synthèse est effectuée à partir du 2-(N-éthylanilino)éthanol, selon le schéma réactionnel suivant :

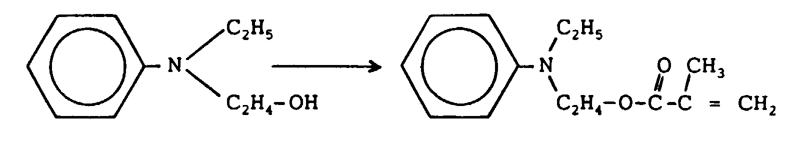

Dans un bicol de 250cm³, équipé d'un réfrigérant et d'une ampoule à brome, on mélange à température ambiante 8,7g (soit 0,0527 mole) de 2-(N-éthyl-anilino)éthanol, 5,33g (soit 0,0527 mole) de triéthylamine, quelques ppm d'hydroquinone, et 150cm³ de chloroforme.

On additionne goutte à goutte 6,05g (soit 0,0579 mole) de chlorure de méthacryloyle (Cl-C(O)-C(CH₃) = CH₂). Une fois l'addition terminée, la solution est chauffée à 45°C pendant 24 heures. On laisse la température du mélange revenir à température ambiante, puis on lave deux fois avec 150 cm³ d'eau. La phase organique est séchée sur du sulfate de sodium et le solvant est chassé. Le mélange alcool-ester est chromatographié sur silice, l'éluant utilisé étant le dichlorométhane. On obtient le 2-(N-éthyl-anilino)éthyl méthacrylate, sous la forme d'une huile jaune, avec un rendement de 65%.

Le produit obtenu a été analysé par RMN ¹H, 200MHz (solvant: CD₃COCD₃)
δ= 1,2ppm(3H,t); 1,95 (3H,s); 3,45 (2H, quad.); 3,7 (2H,t); 4,3 (2H,t); 5,6 (1H,d); 6,1 (1H,s) 6,6-6,8-7,2 ppm (5H aromatiques).

### 2°) Synthèse de l'acide 3-nitro-6-(4'-(N,N-méthacryloxy-2-éthyl,éthylamino) benzène azo)benzoïque.

Cette synthèse comporte d'abord une étape de diazotation de l'acide 3-nitroanthranilique (commercialisé par MAYBRIDGE CO. LIMITED, UK), selon le schéma réactionnel:

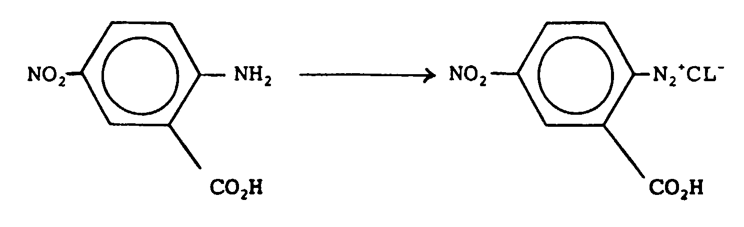

Cette étape est suivie d'une étape de copulation du sel de diazonium obtenu avec le 2-(N-éthyl-anilino)éthyl méthacrylate suivant le schéma réactionnel:

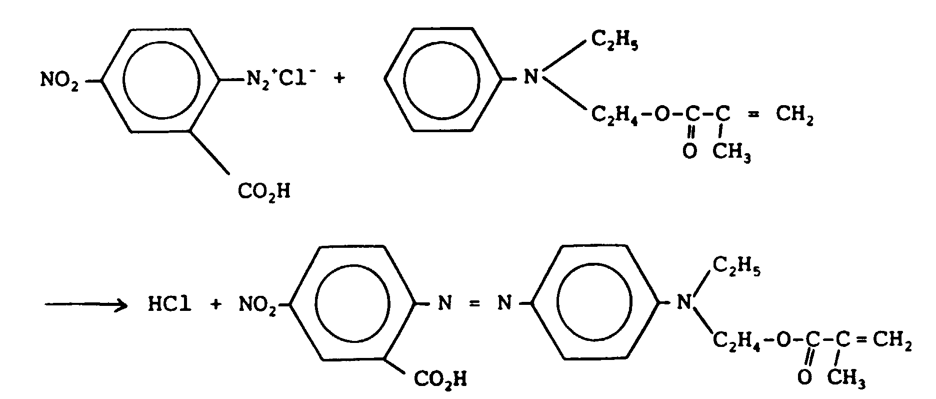

Dans un monocol de 250cm³, on mélange 85cm³ d'une solution de soude 0,3N (soit 0,0257 mole) et 6g (soit 0,0257 mole) d'acide 3-nitroanthranilique. Il est nécessaire de chauffer à 60°C afin de solubiliser l'acide et au besoin, rajouter un peu d'eau. On ajoute alors goutte à goutte, sous une forte agitation mécanique 11cm³ d'une solution d'acide chlorhydrique 10N (soit 0,11 mole). On refroidit la solution lentement à une température comprise entre 0°C et 5°C, puis on ajoute goutte à goutte, sous forte agitation 1,77g (soit 0,0257 mole) de nitrite de sodium dans 5cm³ d'eau. On laisse le mélange sous agitation pendant 2 heures 30 minutes à 5°C, puis on filtre sur büchner.

On additionne alors le filtrat goutte à goutte, à température ambiante, à une solution de 2-(N-éthyl-anilino)éthyl méthacrylate (obtenu par estérification du 2-(N-éthyl anilino) éthanol, vendu par ALDRICH) dans 20cm³ d'éthanol. La couleur vire immédiatement au rouge. Après quelques minutes, il se forme un précipité. Le mélange est laissé sous agitation pendant une nuit, puis la solution est filtrée sur büchner. La fraction solide est lavée avec de l'eau puis avec du pentane. Une chromatographie sur couche mince de silice indique la formation d'un seul produit (éluant CH₂Cl₂-éther: 9/1, produit formé: tâche rouge à Rf=0,49).

Afin d'éliminer les traces d'acide nitroanthranilique restantes, le produit est purifié par une chromatographie sur silice, en utilisant comme éluant un mélange dichlorométhane-éther (9/1). Le rendement est de 49%. Le point de fusion du produit est de 115°C.

Le produit obtenu a été analysé par RMN du proton à 200MHz dans CDCL₃:
δ=1,3 (3H,t); 1,9ppm (3H,s); 3,6ppm (2H, quad); 3,8ppm (2H,t); 4,4ppm (2H,t); 5,6ppm (1H,s); 6,1ppm (1H,s); 6,9-9,1 ppm (7H aromatiques).

Il faut noter le système AB des signaux à 6,8ppm et 7,7ppm du cycle aromatique portant l'amine tertiaire. Ceci montre que la substitution électrophile du sel de diazonium sur ce cycle s'effectue en position 4 par rapport à la fonction amine.

### 3°) Préparation du copolymère réticulable

Ce copolymère est obtenu par polymérisation radicalaire en solution dans du THF (tétrahydrofuranne) à 60°C pendant 36 heures avec de l'AIBN (azobisisobutyronitrile) comme amorceur.

Dans un tube muni d'un rodage vissé, 1,405g (soit 3,3.10⁻³ mole) d'acide 3-nitro-6-(4'-(N,N-méthacryloxy-2-éthyl,éthyl amino) benzène azo) benzoïque, 1,0925 g (soit 7,7.10⁻³ mole) de méthacrylate de glycidyle, et 1,1.10⁻³ mole (soit 0,18g) d'AIBN sont ajoutés à 25ml de THF.

Après un dégazage azote-vide d'une demi heure, le tube est fermé et placé dans un bain thermostaté. L'agitation est assurée par un barreau magnétique. Une fois le temps de polymérisation écoulé, le mélange réactionnel est précipité dans 800ml de méthanol. Le copolymère est repris au dichlorométhane, filtré et séché 24 heures à 40°C sous 20mm de Hg. On récupère ainsi 1,92g de copolymère soit un rendement de 77%. Les masses molaires mesurées avec des étalons PMMA sont Mp= 3400 et Mn=1250. La composition du copolymère déterminée par RMN ¹H et analyses élémentaires est la suivante : le copolymère obtenu contient 26% de motifs comprenant le GONL et 74 % de motifs méthacrylate de glycidyle (en pourcentages molaires).

La température de transition vitreuse est d'environ 70°C.

### 4°) Caractérisation électro-optique et stabilité

Des films de copolymère sont réalisés à la tournette, avec des épaisseurs de l'ordre de 1,2 à 1,5µm sur substrat de verre adéquat (par exemple B K 7). Ils sont soumis à un champ électrique transverse par effet Corona. Les conditions d'orientation des GONL tournent autour de 100°C et 5kV pour la tension Corona (avec une distance de 1cm entre l'électrode et le film) pendant quelques minutes. Le coefficient électro-optique d₃₃, obtenu par les franges de Maker, est de l'ordre de 50pm/V (picomètres par volts) ce qui est une valeur excellente.

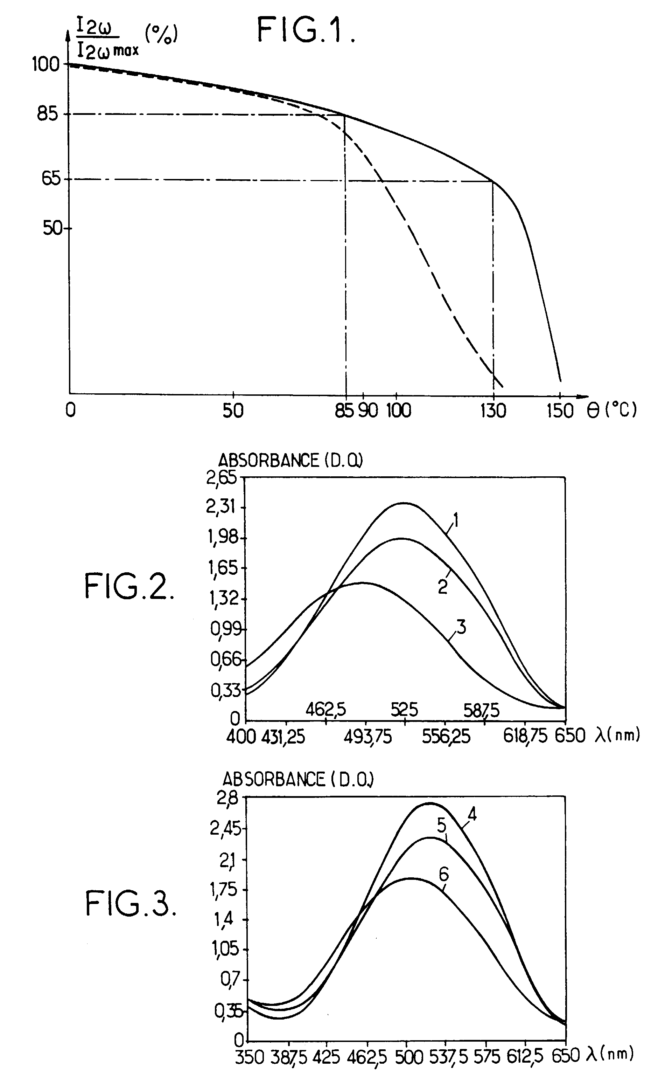

Un tel échantillon n'est pas stable puisque les GONL n'ont pas été réticulés à cette température. Ainsi, comme on peut le voir sur la figure 1 (courbe en pointillés), si on le réchauffe sans application du champ électrique l'intensité du second harmonique émis par effet optique non linéaire s'écroule dès que le film franchit les 90°C.

Par contre, si l'on fait subir consécutivement à l'orientation un recuit à 130°C pendant 3 heures en maintenant le champ électrique, on réticule les GONL en maintenant l'orientation. Ceci procure une stabilité beaucoup plus importante.

Ainsi, comme représenté sur la figure 1 (courbe en trait plein), la génération du second harmonique a baissé seulement de 15% en 1 heure à 85°C, et de 35% à 130°C.

Toutefois, l'orientation est réduite par la réticulation qui force les dipôles à se placer dans des directions qui ne sont pas toujours celles du champ électrique de polarisation. Ainsi, le coefficient électro-optique d₃₃ passe, après réticulation à 130°C pendant 1 heure, de 50pm/V à 20pm/V, ce qui reste une valeur satisfaisante.

### 5°) Mise en évidence de la réticulation entre les groupements carboxyle et époxy, par spectrométrie d'absorption

Les figures 2 et 3 représentent les spectres d'absorption (absorbance en unité de densité optique, en fonction de la longueur d'onde en nanomètres) d'un film du copolymère obtenu comme indiqué au 3° ci-dessus.

Le film est réalisé à la tournette, puis il est uniformisé par recuit à une température proche de sa température de transition vitreuse, puis les chromophores sont orientés par champ électrique Corona légèrement au-dessus de la température de transition vitreuse, et on fige l'orientation par réticulation, en chauffant nettement au-dessus de la température de transition vitreuse, sous champ électrique Corona.

Sur les figures 2 et 3, le spectre d'absorption du film est mesuré à la fin de l'étape de recuit, à la fin de l'étape d'orientation et à la fin de l'étape de réticulation, dans les conditions suivantes :
Figure 2 :
   courbe 1 : après recuit à 75°C pendant 15mn,
   courbe 2 : après orientation sous un champ électrique Corona de 8kV à 1cm du film, à 90°C pendant 30mn,
   courbe 3 : après réticulation sous le champ électrique Corona à 140°C pendant 30mn puis à 160°C pendant 30mn,
Figure 3 :
   courbe 4 : après recuit à 70°C pendant 45mn,
   courbe 5 : après orientation sous un champ électrique Corona de 8kV à 1cm du film, à 90°C pendant 90mn,
   Courbe 6 : après réticulation sous le champ électrique Corona à 130°C pendant 30mn.

Sur les figures 2 et 3, on constate que l'absorbance du film diminue après l'étape d'orientation, ce qui confirme que le chromophores sont bien orientés. De plus, le pic d'absorbance du film est décalé vers les basses longueurs d'ondes, ce qui confirme que les groupements carboxyles ont réagi avec les groupements époxy.

### EXEMPLE II : Greffe d'un groupement photodimérisable sur l'acide 3-nitro-6-(4'-(N,N-méthacryloxy-2-éthyl, éthyl amino) benzène azo) benzoïque

On fait d'abord réagir de l'acide furylacrylique (commercialisé par ALDRICH) et du chlorure de thionyle SOCl₂ en excès à reflux pendant plusieurs heures pour obtenir le chlorure d'acide furylacrylique.

On distille le chlorure de thionyle restant sous 20 Torr à température ambiante, puis on distille le chlorure d'acide furylacrylique à 49°C sous 0,9 mbar.

On obtient ainsi du chlorure d'acide furylacrylique avec un rendement d'environ 80% et une pureté d'environ 99%.

On fait ensuite réagir le chlorure d'acide furylacrylique dissous dans du tétrachlorure de carbone, avec du bromopropanol dans la pyridine, à 50°C pendant 24 heures.

On obtient ainsi le bromopropylester de l'acide furylacrylique : 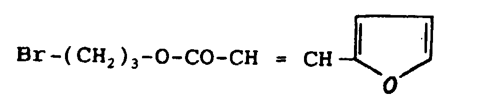 avec un taux de conversion de 85 %.

On fait ensuite réagir le bromopropylester de l'acide furylacrylique avec l'acide 3-nitro-6-(4'-(N,N-méthacryloxy-2-éthyl, éthyl amino) benzène azo)benzoïque, en présence de DBU [1,5-diazabicyclo[5.4.0] undec-7-ène, commercialisé par ALDRICH] et de DMF [diméthylformamide]. On obtient ainsi, avec un rendement supérieur à 30%, le composé : 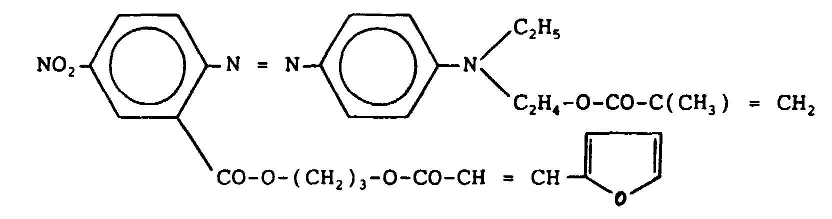 qui peut être polymérisé avec un comonomère portant un groupement 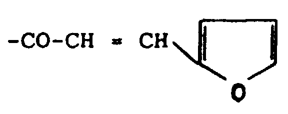 puis réticulé par insolation juste après orientation des chromophores par un champ électrique de polarisation.

## Revendications

1. Matériau réticulable actif en optique non linéaire, comportant un polymère sur lequel sont greffés latéralement des chromophores actifs en optique non linéaire et orientables sous l'effet d'un champ électrique de polarisation, lesdits chromophores comportant un groupe fonctionnel d'ancrage pour stabiliser l'orientation des chromophores par réticulation après leur polarisation par un champ électrique, caractérisé en ce que les chromophores correspondent à la formule (I) suivante : 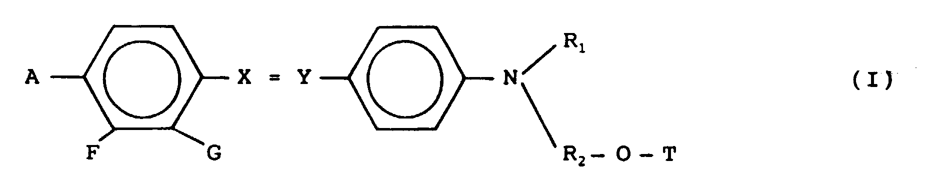 dans laquelle :
- T est le polymère,
- A est un groupement -NO₂, -CN, ou -C(CN)=C(CN)₂,
- R1 est un atome d'hydrogène, un groupement méthyle ou éthyle,
- R2 est un groupement CH₂, C₂H₄, C₃H₆ ou C₄H₈,
- et :
- soit X = Y est un groupement azoïque, F étant un atome d'hydrogène et G étant un groupement -C(O)-O-W ou un groupement -C(O)-O-(CH₂)ₙ-O-W, n étant un entier compris entre 2 et 6 et W étant un atome d'hydrogène ou un groupement dimérisable, G constituant alors le groupe fonctionnel d'ancrage susmentionné,
- soit X = Y est un groupement azométhine, F étant un groupement -O-W ou -R₃-O-W, où W est un atome d'hydrogène ou un groupement dimérisable, R₃ étant un groupement -O-C(O)-R₄- ou -O-R₄-, où R₄ est un alkyle C₁ à C₆, et G étant un atome d'hydrogène, F constituant alors ledit groupe fonctionnel d'ancrage,
- soit X = Y est un groupement azométhine, G étant un groupement -O-W ou -R₃-O-W, où W est un atome d'hydrogène ou un groupement dimérisable, R₃ étant un groupement -O-C(O)-R₄- ou -O-R₄-, où R₄ est un alkyle C₁ à C₆ et F étant un atome d'hydrogène, G constituant alors ledit groupe fonctionnel d'ancrage,
ledit matériau comportant en outre au moins un groupe fonctionnel d'ancrage complémentaire, susceptible de se lier au groupe fonctionnel d'ancrage porté par le chromophore lors de la réticulation.

2. Matériau selon la revendication 1, dans lequel X = Y est un groupement azométhine et F est un groupement -R₃-O-W, R₃ étant un groupement -O-R₄.

3. Matériau selon la revendication 1 dans lequel X = Y est un groupement azométhine et G est un groupement -R₃-O-W, R₃ étant un groupement -O-C(O)-R₄.

4. Matériau selon l'une quelconque des revendications précédentes, dans lequel le chromophore est compris dans un motif du polymère qui est choisi parmi les acrylates, les méthacrylates et les styréniques.

5. Matériau selon l'une quelconque des revendications 1 à 3, dans lequel le chromophore est compris dans un motif du polymère qui est choisi parmi les α-haloacrylates, les cétones et les éthers vinyliques.

6. Matériau selon l'une quelconque des revendications précédentes, dans lequel le polymère comporte, outre le motif qui comprend le chromophore, un motif additionnel, le motif qui comprend le chromophore représentant une proportion molaire comprise entre 10 et 50 % des motifs du polymère.

7. Matériau selon la revendication 6, dans lequel le motif additionnel est choisi dans la famille des acrylates ou des méthacrylates, dans la famille des styréniques, dans la famille des esters vinyliques, dans la famille des éthers vinyliques ou dans la famille des carbonates vinyliques.

8. Matériau selon l'une quelconque des revendications 6 et 7, dans lequel le groupe fonctionnel d'ancrage complémentaire est compris dans le motif additionnel.

9. Matériau selon l'une quelconque des revendications 1 à 7, dans lequel le groupe fonctionnel d'ancrage complémentaire est porté par des molécules additionnelles, mélangées intimement au polymère qui porte le chromophore, ces molécules additionnelles étant des molécules polymérisées ayant un motif qui inclut le groupe fonctionnel d'ancrage complémentaire.

10. Matériau selon l'une quelconque des revendications 1 à 7, dans lequel le groupe fonctionnel d'ancrage complémentaire est porté par des molécules additionnelles, mélangées intimement au polymère qui porte le chromophore, ces molécules additionnelles portant chacune deux groupes fonctionnels d'ancrage complémentaires qui sont identiques, greffés sur un radical alkyle ou aryle C₁ à C₁₀, chaque groupe fonctionnel d'ancrage pouvant réagir avec le groupe fonctionnel d'ancrage d'un chromophore.

11. Matériau selon l'une quelconque des revendications précédentes, dans lequel G est un groupement -COOH qui constitue le groupe fonctionnel d'ancrage du chromophore, et le groupe fonctionnel d'ancrage complémentaire est un groupement isocyanate, époxyde, hydroxyle, ou -SH.

12. Matériau selon la revendication 11, dans lequel le chromophore est compris dans un motif du polymère qui présente la formule (II) suivante :

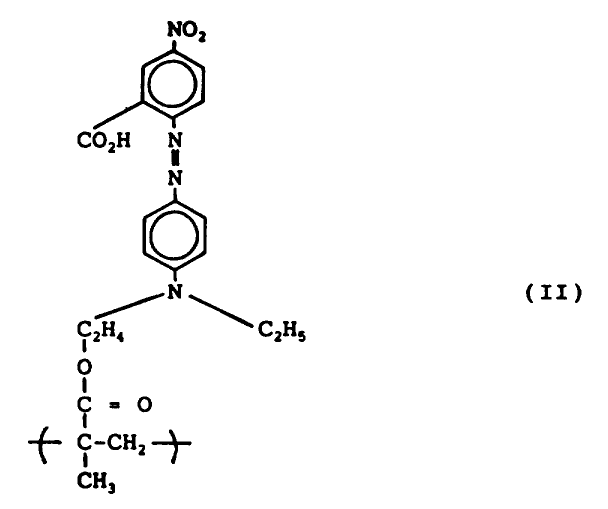

13. Matériau selon l'une quelconque des revendications 11 et 12, dans lequel le polymère comporte, outre le motif qui inclut le chromophore, au moins un motif qui présente la formule (III) suivante : 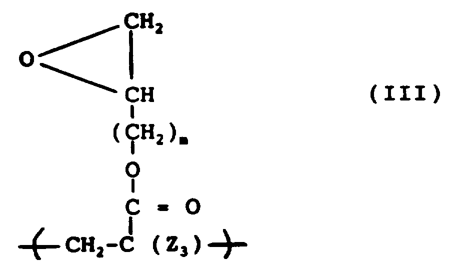 où :
- Z₃ est un atome d'hydrogène, de fluor, ou de chlore ou un groupement méthyle, et
- m est un entier compris entre 1 et 6.

14. Matériau selon la revendication 13, dans lequel Z₃ est un groupement méthyle et m est égal à 1.

15. Matériau selon l'une quelconque des revendications 1 à 10, dans lequel l'un des groupements F et G comporte une fonction alcool et constitue le groupe fonctionnel d'ancrage du chromophore, le groupe fonctionnel d'ancrage complémentaire étant un groupement isocyanate, COOH, ou chlorure d'acide.

16. Matériau selon la revendication 15, dans lequel le polymère comporte, outre le motif qui inclut le chromophore, au moins un motif qui présente la formule (IV) suivante :

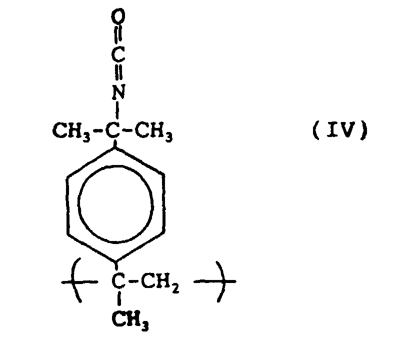

17. Matériau selon l'une quelconque des revendications 1 à 10 dans lequel W est un groupement dimérisable choisi dans le groupe comprenant :
-C(O)-C(Z₂) = CH₂ 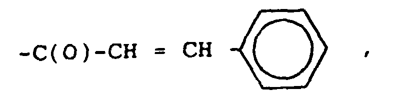 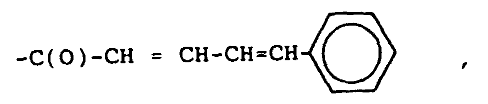 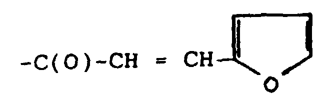
et 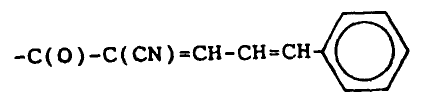 Z₂ étant un atome d'hydrogène, de fluor ou de chlore ou un groupement méthyle, et le groupe fonctionnel d'ancrage complémentaire comportant également un groupement fonctionnel dimérisable choisi dans le même groupe que W.

18. Matériau selon la revendication 17, dans lequel les groupements dimérisables du groupe fonctionnel d'ancrage porté par le chromophore et du groupe fonctionnel d'ancrage complémentaire, sont identiques.

19. Matériau selon la revendication 18, dans lequel les groupes fonctionnels d'ancrage portés par les chromophores constituent au moins une partie des groupes fonctionnels d'ancrage complémentaires, la réticulation se faisant au moins en partie par dimérisation entre chromophores.

20. Matériau selon l'une quelconque des revendications 17 à 19, dans lequel le motif incluant le chromophore a la formule (V) suivante :

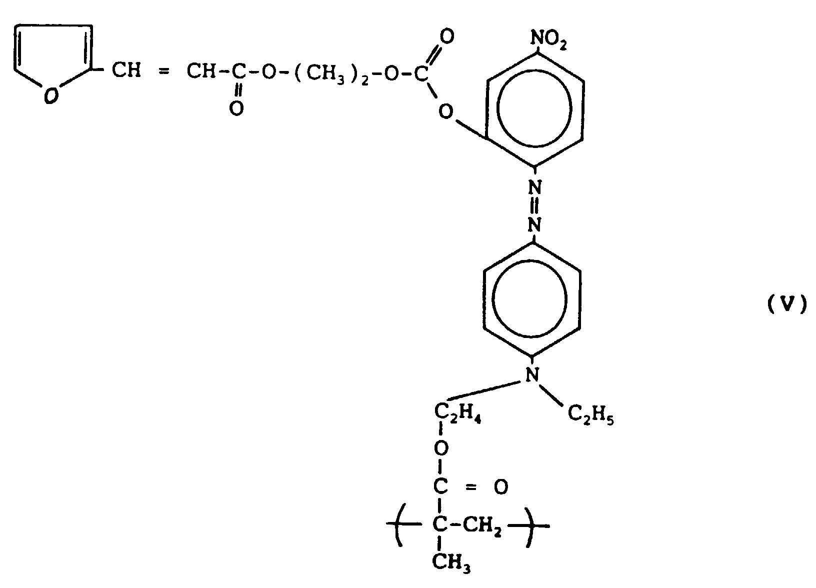

21. Matériau réticulé actif en optique non linéaire, obtenu par réticulation d'un matériau selon l'une quelconque des revendications précédentes, après polarisation de ses chromophores par un champ électrique, la réticulation étant obtenue par liaison chimique entre le groupe fonctionnel d'ancrage porté par le chromophore, et le groupe fonctionnel d'ancrage complémentaire.

22. Utilisation d'un matériau polymérisé réticulé selon la revendication 21, dans un composant optique.

23. Monomère actif en optique non linéaire, polymérisable et comportant un groupe fonctionnel d'ancrage, caractérisé en ce qu'il correspond à la formule (VI) suivante : 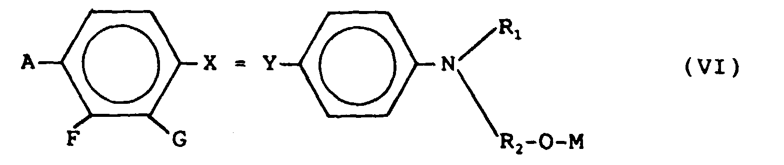 dans laquelle
- A est un groupement NO₂, CN, ou C(CN)=C(CN)₂
- R1 est un atome d'hydrogène, un groupement méthyle ou un groupement éthyle,
- R2 est un groupement CH₂, C₂H₄, C₃H₆ ou C₄H₈,
- M est choisi dans la famille des radicaux acryliques, méthacryliques et des styréniques,
- et :
- soit X = Y est un groupement azoïque, F étant un atome d'hydrogène et G étant un groupement -C(O)-O-W ou un groupement -C(O)-O-(CH₂)ₙ-O-W, n étant un entier compris entre 2 et 6 et W étant un atome d'hydrogène ou un groupement dimérisable, G constituant alors le groupe fonctionnel d'ancrage susmentionné,
- soit X = Y est un groupement azométhine, F étant un groupement -O-W ou -R₃-O-W, où W est un atome d'hydrogène ou un groupement dimérisable, R₃ étant un groupement -O-C(O)-R₄- ou -O-R₄-, où R₄ est un alkyle C₁ à C₆, et G étant un atome d'hydrogène, F constituant alors ledit groupe fonctionnel d'ancrage,
- soit X = Y est un groupement azométhine, G étant un groupement -O-W ou -R₃-O-W, où W est un atome d'hydrogène ou un groupement dimérisable, R₃ étant un groupement -O-C(O)-R₄- ou -O-R₄-, où R₄ est un alkyle C₁ à C₆, et F étant un atome d'hydrogène, G constituant alors ledit groupe fonctionnel d'ancrage.

24. Monomère selon la revendication 23, dans lequel X = Y est un groupement azométhine et F est un groupement -R₃-O-W, R₃ étant un groupement -O-R₄.

25. Monomère selon la revendication 23, dans lequel X = Y est un groupement azométhine et G est un groupement -R₃-O-W, R₃ étant un groupement -O-C(O)-R₄.

26. Monomère selon l'une quelconque des revendications 23 à 25, dans lequel W est un groupement dimérisable choisi dans le groupe comprenant
-C(O)-C(Z₂) = CH₂ 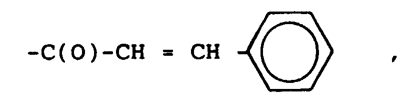 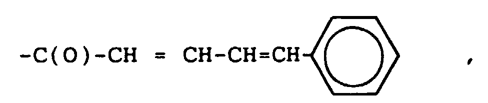 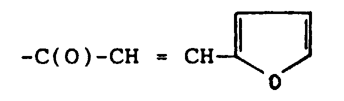
et 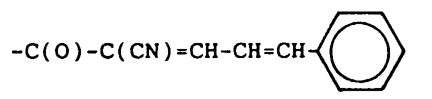 Z₂ étant un atome d'hydrogène, de fluor ou de chlore ou un groupement méthyle, et le groupe fonctionnel d'ancrage complémentaire comporte également un groupement fonctionnel dimérisable choisi dans le même groupe.

27. Monomère selon la revendication 23, de formule (VII) suivante :

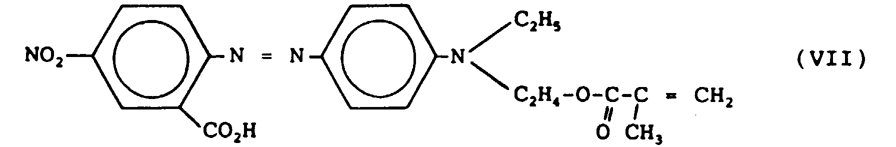

28. Procédé d'obtention d'un monomère actif en optique non linéaire selon la revendication 23, dans lequel X = Y est un groupement azoïque, à partir des composés : 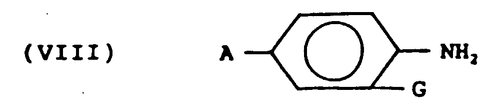 et 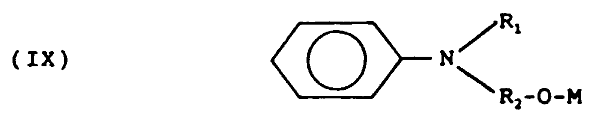 caractérisé en ce qu'il comporte une étape de diazotation du composé (VIII) pour obtenir un sel de diazonium de ce composé, puis une étape de copulation entre ledit sel de diazonium et le composé (IX).

29. Procédé selon la revendication 28, dans lequel M est un groupement appartenant à la famille des radicaux acryliques ou méthacryliques, et dans lequel le composé (IX) est préparé par estérification entre le composé (X) : 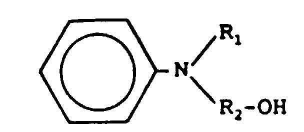 et l'acide HO-M ou son chlorure d'acide.

30. Procédé d'obtention d'un matériau selon l'une quelconque des revendications 1 à 21, dans lequel G est un groupement -C(O)-O-(CH₂)ₙ-OH, et dans lequel l'obtention du groupement G comporte une étape d'estérification d'un composé Br-(CH₂)ₙ-OH sur un groupement -COOH situé initialement à la place du groupement G dans le chromophore.

31. Procédé d'obtention d'un matériau selon l'une des revendications 1 à 21, dans lequel G est un groupement -C(O)-O-(CH₂)ₙ-O-W et -O-W est un groupement carboxylique dimérisable, comportant une étape d'estérification entre l'alcool
Br-(CH₂)ₙ-OH
et le chlorure d'acide Cl-W pour obtenir le composé (XI) :
Br-(CH₂)ₙ-O-W,
puis une étape d'estérification entre le composé (XI) et un groupement -COOH situé initialement à la place du groupement G dans le chromophore.

## Claims

1. A crosslinkable material that is active in non-linear optics and that contains a polymer on which are laterally grafted chromophores that are active in non-linear optics and that may be oriented under the effect of a polarizing electric field, the said chromophores containing an anchoring functional group for stabilizing the orientation of the chromophores by crosslinking after their polarization by an electric field, wherein the chromophores correspond to the following formula (I): 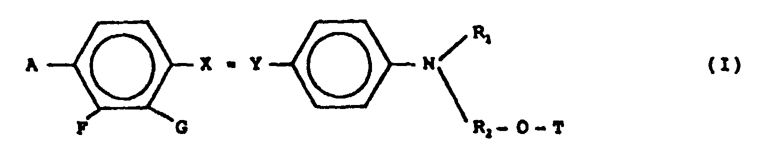 in which:
- T is the polymer,
- A is an -NO₂, -CN or -C(CN)=C(CN)₂ group,
- R₁ is a hydrogen atom or a methyl or ethyl group,
- R₂ is a CH₂, C₂H₄, C₃H₆, or C₄H₈ group,
- and:
- either X = Y is an azo group, F being a hydrogen atom and G being a group -C(O)-O-W or a group -C(O)-O-(CH₂)ₙ-O-W, n being an integer between 2 and 6 and W being a hydrogen atom or a dimerizable group, G then constituting the abovementioned anchoring functional group,
- or X - Y is an azomethine group, F being a group -O-W or -R₃-O-W, where W is a hydrogen atom or a dimerizable group, R₃ being a group -O-C(O)-R₄- or -O-R₄-, where R₄ is a C₁ to C₆ alkyl, and G being a hydrogen atom, F then constituting the said anchoring functional group,
- or X = Y is an azomethine group, G being a group -O-W or -R₃-O-W, where W is a hydrogen atom or a dimerizable group, R, being a group -O-C(O)-R₄- or -O-R₄-, where R₄ is a C₁ to C₆ alkyl, and F being a hydrogen atom, G then constituting the said anchoring functional group,
the said material additionally containing at least one complementary anchoring functional group capable of attaching itself to the anchoring functional group carried by the chromophore during the crosslinking.

2. The material as claimed in claim 1, wherein X = Y is an azomethine group and F is a group -R₃-O-W, R₃ being a group -O-R₄.

3. The material as claimed in claim 1, wherein X = Y is an azomethine group and G is a group -R₃-O-W, R₃ being a group -O-C(O)-R₄.

4. The material as claimed in any one of the preceding claims, wherein the chromophore is included in a unit of the polymer which is chosen from acrylates, methacrylates and styrenes.

5. The material as claimed in any one of the preceding claims, wherein the chromophore is included in a unit of the polymer which is chosen from α-haloacrylates, ketones and vinyl ethers.

6. The material as claimed in any one of claims 1-3, wherein the polymer contains, besides the unit which comprises the chromophore, an additional unit, the unit which comprises the chromophore representing a molar proportion of between 10 and 50% of the units of the polymer.

7. The material as claimed in claim 6, wherein the additional unit is chosen from the acrylate or methacrylate family, from the styrene family, from the vinyl ester family, from the vinyl ether family or from the vinyl carbonate family.

8. The material as claimed in any one of claims 6 and 7, wherein the complementary anchoring functional group is included in the additional unit.

9. The material as claimed in any one of claims 1-7, wherein the complementary anchoring functional group is carried by additional molecules, intimately mixed with the polymer which carries the chromophore, these additional molecules being polymerized molecules having a unit which includes the complementary anchoring functional group.

10. The material as claimed in any one of claims 1-7, wherein the complementary anchoring functional group is carried by additional molecules, intimately mixed with the polymer which carries the chromophore, these additional molecules each carrying two complementary anchoring functional groups which are identical grafted onto a C₁ to C₁₀ aryl or alkyl radical, it being possible for each anchoring functional group to react with the anchoring functional group of a chromophore.

11. The material as claimed in any one of the preceding claims, wherein G is a -COOH group which constitutes the anchoring functional group of the chromophore and the complementary anchoring functional group is an isocyanate, epoxide, hydroxyl or -SH group.

12. The material as claimed in claim 11, wherein the chromophore is included in a unit of the polymer which has the following formula (II):

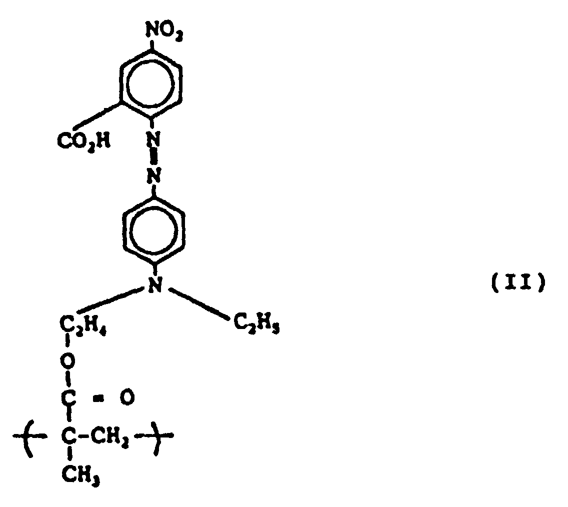

13. The material as claimed in any one of claims 11 and 12, wherein the polymer contains, besides the unit which includes the chromophore, at least one unit which has the following formula (III): 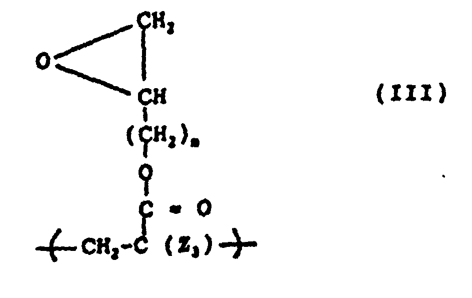 where :
- Z₃ is a hydrogen, fluorine or chlorine atom or a methyl group, and
- m is an integer between 1 and 6.

14. The material as claimed in claim 13, wherein Z₃ is a methyl group and m is equal to 1.

15. The material as claimed in any one of claims 1-10, wherein one of the groups F and G contains an alcohol function and constitutes the anchoring functional group of the chromophore, the complementary anchoring functional group being an isocyanate, -COOH or acid chloride group.

16. The material as claimed in claim 15, wherein the polymer contains, besides the unit which includes the chromophore, at least one unit which has the following formula (IV):

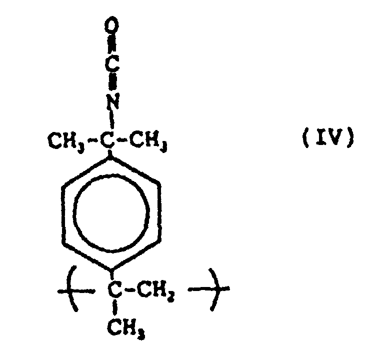

17. The material as claimed in any one of claims 1-10, wherein W is a dimerizable group chosen from the group comprising:
-C(O)-C(Z₂) = CH₂ 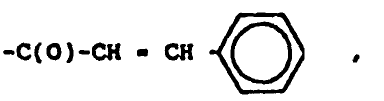 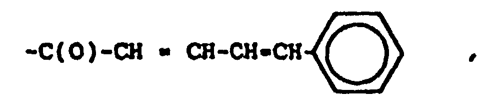 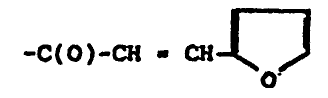
and 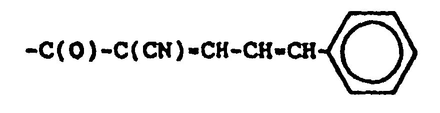 Z₂ being a hydrogen, fluorine or chlorine atom or a methyl group, and the complementary anchoring functional group also containing a dimerizable functional group chosen from the same group as W.

18. The material as claimed in claim 17, wherein the dimerizable groups of the anchoring functional group carried by the chromophore and of the complementary anchoring functional group are identical.

19. The material as claimed in claim 18, wherein the anchoring functional groups carried by the chromophores constitute at least some of the complementary anchoring functional groups, the crosslinking being effected at least partly by dimerization between chromophores.

20. The material as claimed in any one of claims 17-19, wherein the unit including the chromophore has the following formula (V):

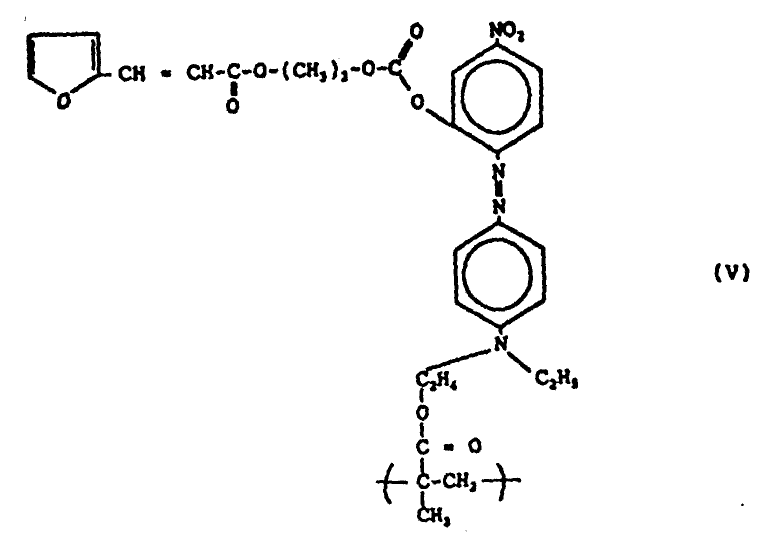

21. A crosslinked material that is active in non-linear optics, obtained by crosslinking the material as claimed in any one of the preceding claims, after polarization of its chromophores by an electric field, the crosslinking being obtained by chemical bonding between the anchoring functional group carried by the chromophore and the complementary anchoring functional group.

22. Use of a crosslinked polymerized material as claimed in claim 21, in an optical component.

23. A polymerizable monomer that is active in non-linear optics and that contains an anchoring functional group, this monomer corresponding to the following formula (VI): 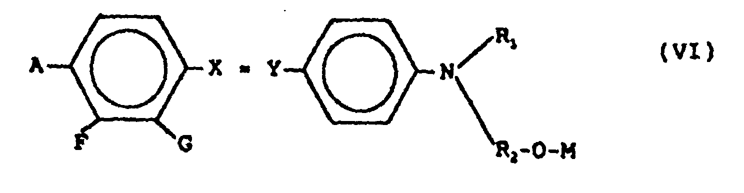 in which
- A is an NO₂, CN or C(CN)=C(CN)₂ group
- R₁ is a hydrogen atom, a methyl group or an ethyl group,
- R₂ is a CH₂, C₂H₄, C₃H₆, or C₄H₈ group,
- M is chosen from the family of acrylic, methacrylic and styrene radicals,
- and:
- either X = Y is an azo group, F being a hydrogen atom and G being a group -C(O)-O-W or a group -C(O)-O-(CH₂)ₙ-O-W, n being an integer between 2 and 6 and W being a hydrogen atom or a dimerizable group, G then constituting the above-mentioned anchoring functional group,
- or X = Y is an azomethine group, F being a group -O-W or -R₃-O-W, where W is a hydrogen atom or a dimerizable group, R₃ being a group -O-C(O)-R₄- or -O-R₄-, where R₄ is a C₁ to C₆ alkyl, and G being a hydrogen atom, F then constituting the said anchoring functional group,
- or X = Y is an azomethine group, G being a group -O-W or -R₃-O-W, where W is a hydrogen atom or a dimerizable group, R₃ being a group -O-C(O)-R₄- or -O-R₄-, where R₄ is a C₁ to C₆ alkyl, and F being a hydrogen atom, G then constituting the said anchoring functional group.

24. The monomer as claimed in claim 23, wherein X = Y is an azomethine group and F is a group -R₃-O-W, R₃ being a group -O-R₄.

25. The monomer as claimed in claim 23, wherein X = Y is an azomethine group and G is a group -R₃-O-W, R₃ being a group -O-C(O)-R₄.

26. The monomer as claimed in any one of claims 23-25, wherein W is a dimerizable group chosen from the group comprising:
-C(O)-C(Z₂) = CH₂ 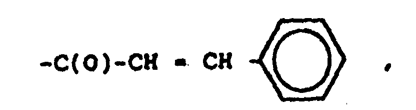 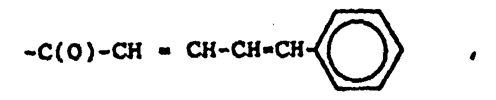 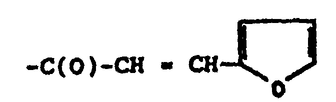
and 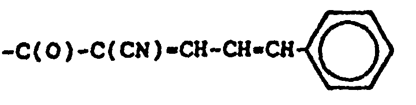 Z₂ being a hydrogen, fluorine or chlorine atom or a methyl group, and the complementary anchoring functional group also contains a dimerizable functional group chosen from the same group.

27. The monomer as claimed in claim 23, of following formula (VII):

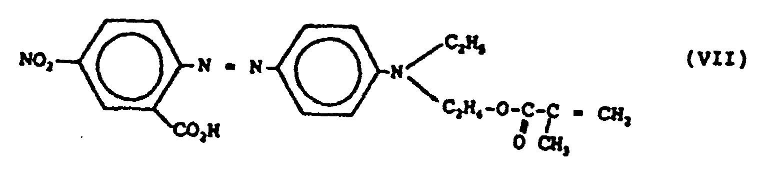

28. A process for obtaining the monomer that is active in non-linear optics as claimed in claim 23, in which X = Y is an azo group, from the compounds: 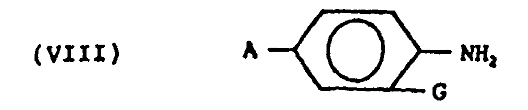 and 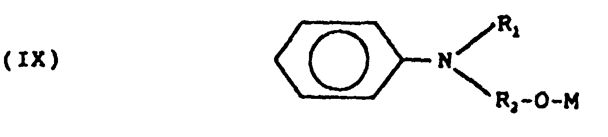 which comprises a step of diazotization of the compound (VIII) in order to obtain a diazonium salt of this compound, followed by a coupling step between the said diazonium salt and the compound (IX).

29. The process as claimed in claim 28, wherein M is a group belonging to the family of the acrylic or methacrylic radicals and wherin the compound (IX) is prepared by esterification between the compound (X): 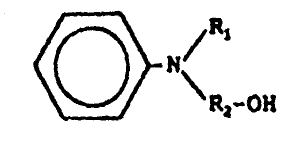 and the acid HO-M or its acid chloride.

30. A process for obtaining the material as claimed in any one of claims 1-21, in which G is a group -C(O)-O-(CH₂)ₙ-OH, which comprises the production of the group G by an esterification step of a compound Br-(CH₂)ₙ-OH with a -COOH group located initially in place of the group G in the chromophore.

31. A process for obtaining the material as claimed in any one of claims 1-21, in which G is a group -C(O)-O-(CH₂)ₙ-O-W and -O-W is a dimerizable carboxyl group, which comprises an esterification step between the alcohol
Br-(CH₂)ₙ-OH
and the acid chloride Cl-W in order to obtain the compound (XI):
Br-(CH₂)ₙ-O-W,
followed by an esterification step between the compound (XI) and a -COOH group located initially in place of the group G in the chromophore.

## Patentansprüche

1. Vernetzbares Material mit nicht-linearen optischen Eigenschaften, umfassend ein Polymer, an dem seitlich Chromophore mit nicht-linearen optischen Eigenschaften angebracht sind, die unter der Wirkung eines elektrischen Polarisierungsfeldes orientierbar sind, wobei auf den Chromophoren eine funktionelle Ankergruppe vorhanden ist um die Orientierung der Chromophore mittels Vernetzung nach deren Polarisierung durch ein elektrisches Feld zu stabilisieren, dadurch gekennzeichnet, daß die Chromophore der nachfolgenden Formel (I): 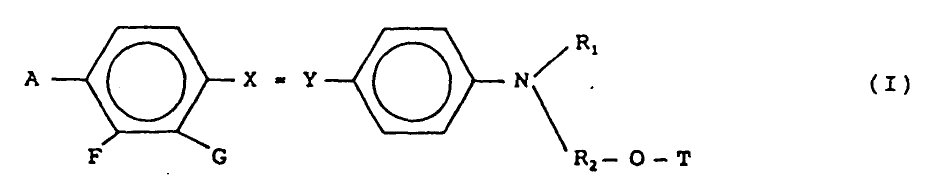 entsprechen, wobei:
- T das Polymer ist,
- A eine -NO₂, -CN oder -C(CN)=C(CN)₂ Gruppe ist,
- R₁ ein Wasserstoffatom, eine Methyl- oder Ethylgruppe ist,
- R₂ eine CH₂, C₂H₄, C₃H₆ oder C₄H₈ Gruppe ist,
- und:
- wenn X = Y eine Azogruppe ist, F ein Wasserstoffatom ist und G eine -C(O)-O-W Gruppe oder eine -C(O)-O-(CH₂)ₙ-O-W Gruppe ist, wobei n eine ganze Zahl zwischen 2 und 6 ist und W ein Wasserstoffatom oder eine dimerisierbare Gruppe ist, somit G die vorstehend genannte funktionelle Ankergruppe darstellt,
- wenn X = Y eine Azomethingruppe ist, F eine -O-W oder eine R₃-O-W Gruppe ist, wobei W ein Wasserstoffatom oder eine dimerisierbare Gruppe ist, R₃ eine -O-C(O)-R₄- oder -O-R₄- Gruppe ist, wobei R₄ ein C₁ bis C₆ Alkyl ist, und G ein Wasserstoffatom ist, somit F die funktionelle Ankergruppe darstellt,
- wenn X = Y eine Azomethingruppe ist, G eine -O-W oder eine R₃-O-W Gruppe ist, wobei W ein Wasserstoffatom oder eine dimerisierbare Gruppe ist, R₃ eine -O-C(O)-R₄- oder -O-R₄- Gruppe ist, wobei R₄ ein C₁ bis C₆ Alkyl ist, und F ein Wasserstoffatom ist, somit G die funktionelle Ankergruppe darstellt,
wobei das Material weiterhin mindestens eine komplementäre Ankergruppe umfaßt, welche zur Bindung an die auf dem Chromophor vorhandene Ankergruppe bei der Vernetzung geeignet ist.

2. Material nach Anspruch 1, wobei X = Y eine Azomethingruppe ist und F eine R₃-O-W Gruppe ist, wobei R₃ eine -O-R₄- Gruppe ist.

3. Material nach Anspruch 1, wobei X = Y eine Azomethingruppe ist und G eine R₃-O-W Gruppe ist, wobei R₃ eine -O-C(O)-R₄- Gruppe ist.

4. Material nach einem der vorhergehenden Ansprüche, wobei das Chromophor in einer Polymereinheit, ausgewählt aus den Acrylaten, den Methacrylaten und den Styrolen enthalten ist.

5. Material nach einem der Ansprüche 1 bis 3, wobei das Chromophor in einer Polymereinheit, ausgewählt aus den α-Halogenacrylaten, den Ketonen und den Vinylethern enthalten ist.

6. Material nach einem der vorhergehenden Ansprüche, wobei das Polymer neben der das Chromophor umfassenden Einheit eine weitere Einheit umfaßt, wobei die das Chromophor umfassende Einheit einen molaren Anteil umfassend zwischen 10 und 50 % der Polymereinheiten aufweist.

7. Material nach Anspruch 6, wobei die weitere Einheit ausgewählt ist aus der Gruppe der Acrylate oder der Methacrylate, aus der Gruppe der Styrole, aus der Gruppe der Vinylester, aus der Gruppe der Vinylether oder aus der Gruppe der Vinylcarbonate.

8. Material nach einem der Ansprüche 6 und 7, wobei die komplementäre funktionelle Ankergruppe in der weiteren Einheit enthalten ist.

9. Material nach einem der Ansprüche 1 bis 7, wobei die komplementäre funktionelle Ankergruppe auf zusätzlichen Molekülen vorhanden ist, die mit dem das Chromophor enthaltenden Polymer innig vermischt sind, wobei diese zusätzlichen Moleküle Polymermoleküle mit einer die komplementäre funktionelle Ankergruppe umfassenden Einheit sind.

10. Material nach einem der Ansprüche 1 bis 7, wobei die komplementäre funktionelle Ankergruppe auf zusätzlichen Molekülen vorhanden ist, die mit dem das Chromophor enthaltenden Polymer innig vermischt sind, wobei auf diesen zusätzlichen Molekülen jeweils zwei identische komplementäre funktionelle Ankergruppen, die an einem C₁ bis C₁₀ Alkyl- oder Arylrest angebracht sind, vorhanden sind, wobei jede funktionelle Ankergruppe mit der funktionellen Ankergruppe eines Chromophors reagieren kann.

11. Material nach einem der vorhergehenden Ansprüche, wobei G eine -COOH Gruppe ist, welche die funktionelle Ankergruppe des Chromophors darstellt, und die komplementäre funktionelle Ankergruppe eine Isocyanat, Epoxid, Hydroxyl oder -SH Gruppe ist.

12. Material nach Anspruch 11, wobei das Chromophor in einer Einheit des Polymers vorhanden ist, welche durch die nachfolgende Formel (II) dargestellt wird:

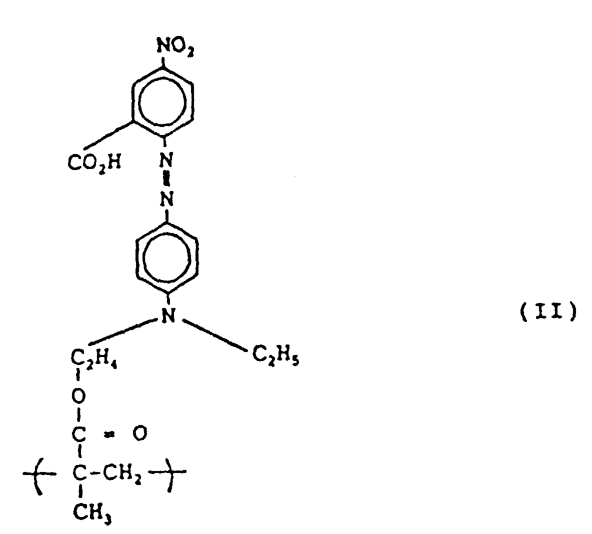

13. Material nach einem der Ansprüche 11 und 12, wobei das Polymer neben der das Chromophor umfassenden Einheit mindestens eine Einheit umfaßt, welche durch die nachfolgende Formel (III): 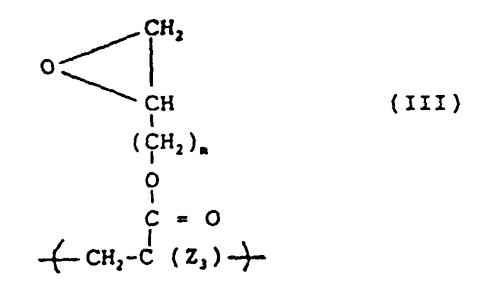 dargestellt ist, wobei:
- Z₃ ein Wasserstoff-, Fluor- oder Chloratom oder eine Methylgruppe ist, und
- m eine ganze Zahl zwischen 1 und 6 ist.

14. Material nach Anspruch 13, wobei Z₃ eine Methylgruppe ist und m gleich 1 ist.

15. Material nach einem der Ansprüche 1 bis 10, wobei eine der Gruppen F und G eine Alkohol funktion umfaßt und die funktionelle Ankergruppe des Chromophors darstellt, wobei die komplementäre funktionelle Ankergruppe eine Isocyanat-, COOH- oder Säurechloridgruppe ist.

16. Material nach Anspruch 15, wobei das Polymer neben der das Chromophor umfassenden Einheit mindestens eine Einheit umfaßt, welche durch die nachfolgende Formel (IV) dargestellt ist:

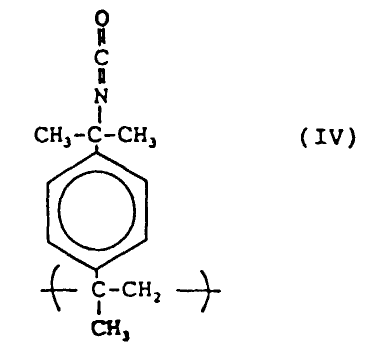

17. Material nach einem der Ansprüche 1 bis 10, wobei W eine dimerisierbare Gruppe ist, ausgewählt aus der Gruppe umfassend:
-C(O)-C(Z₂) = CH₂ 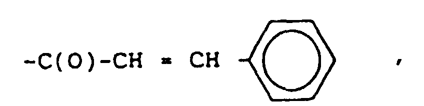 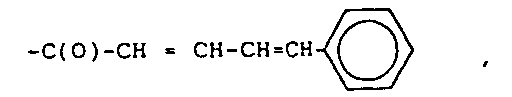 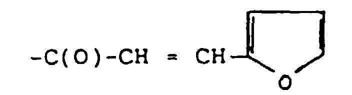
und 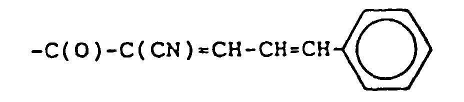 wobei Z₂ ein Wasserstoff-, Fluor- oder Chloratom oder eine Methylgruppe ist und die komplementäre funktionelle Ankergruppe ebenfalls eine dimerisierbare funktionelle Gruppe umfaßt, welche aus der gleichen Gruppe wie W ausgewählt ist.

18. Material nach Anspruch 17, wobei die dimerisierbaren Gruppen der auf dem Chromophor vorhandenen funktionellen Ankergruppe und der komplementären funktionellen Ankergruppe identisch sind.

19. Material nach Anspruch 18, wobei die auf den Chromophoren vorhandenen funktionellen Ankergruppen mindestens einen Teil der komplementären funktionellen Ankergruppen ausmachen, wobei man die Vernetzung mindestens teilweise durch Dimerisation zwischen Chromophoren bewerkstelligt.

20. Material nach einem der Ansprüche 17 bis 19, wobei die das Chromophor umfassende Einheit der nachfolgenden Formel (V) entspricht:

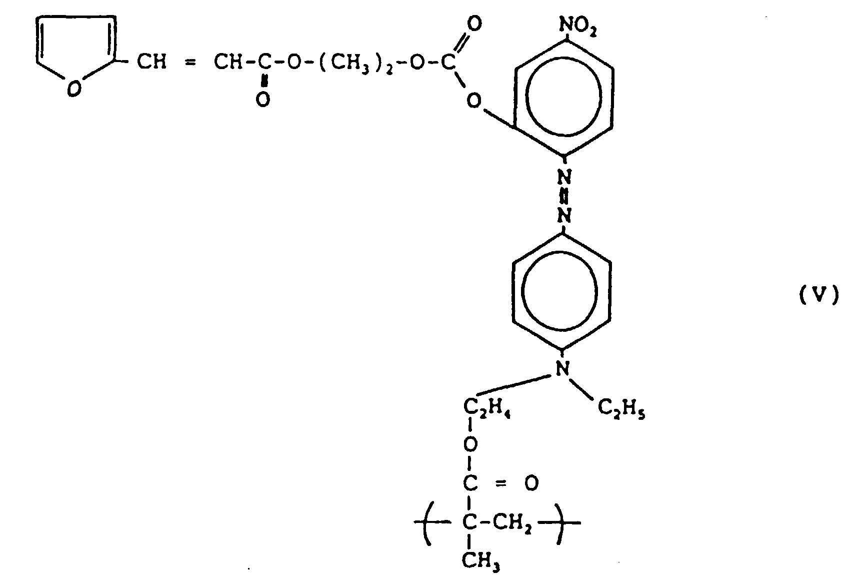

21. Vernetztes Material mit nicht-linearen optischen Eigenschaften, erhalten durch Vernetzung eines Materials nach einem der vorhergehenden Ansprüche nach Polarisierung dessen Chromophoren mittels eines elektrischen Feldes, wobei die Vernetzung erhalten wird durch chemische Bindung zwischen einer auf dem Chromophor vorhandenen funktionellen Ankergruppe und einer komplementären funktionellen Ankergruppe.

22. Verwendung eines vernetzten Polymermaterials nach Anspruch 21 in einem optischen Bauteil.

23. Polymerisierbares Monomer mit nicht-linearen optischen Eigenschaften, das eine funktionelle Ankergruppe umfaßt, dadurch gekennzeichnet, daß es der nachfolgenden Formel (VI): 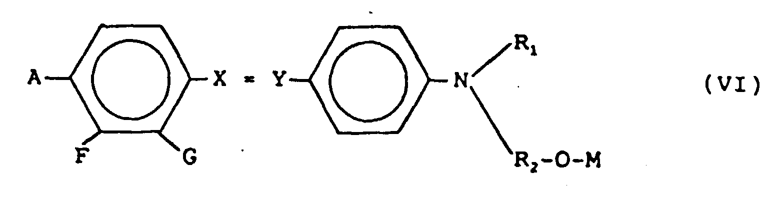 entspricht, wobei:
- A eine -NO₂, -CN oder -C(CN)=C(CN)₂ Gruppe ist,
- R₁ ein Wasserstoffatom, eine Methylgruppe oder eine Ethylgruppe ist,
- R₂ eine CH₂, C₂H₄, C₃H₆ oder C₄H₈ Gruppe ist,
- M ausgewählt ist aus der Gruppe Acryl-, Methacryl- und Styrolreste
- und:
- wenn X = Y eine Azogruppe ist, F ein Wasserstoffatom ist und G eine -C(O)-O-W Gruppe oder eine -C(O)-O-(CH₂)ₙ-O-W Gruppe ist, wobei n eine ganze Zahl zwischen 2 und 6 ist und W ein Wasserstoffatom oder eine dimerisierbare Gruppe ist, somit G die vorstehend genannte funktionelle Ankergruppe darstellt,
- wenn X = Y eine Azomethingruppe ist, F eine -O-W oder eine R₃-O-W Gruppe ist, wobei W ein Wasserstoffatom oder eine dimerisierbare Gruppe ist, R₃ eine -O-C(O)-R₄- oder -O-R₄- Gruppe ist, wobei R₄ ein C₁ bis C₆ Alkyl ist, und G ein Wasserstoffatom ist, somit F die funktionelle Ankergruppe darstellt,
- wenn X = Y eine Azomethingruppe ist, G eine -O-W oder eine R₃-O-W Gruppe ist, wobei W ein Wasserstoffatom oder eine dimerisierbare Gruppe ist, R₃ eine -O-C(O)-R₄- oder -O-R₄- Gruppe ist, wobei R₄ ein C₁ bis C₆ Alkyl ist, und F ein Wasserstoffatom ist, somit G die funktionelle Ankergruppe darstellt.

24. Monomer nach Anspruch 23, wobei X = Y eine Azomethingruppe ist'und F eine R₃-O-W Gruppe ist, wobei R₃ eine -O-R₄- Gruppe ist.

25. Monomer nach Anspruch 23, wobei X = Y eine Azomethingruppe ist und G eine R₃-O-W Gruppe ist, wobei R₃ eine -O-C(O)-R₄- Gruppe ist.

26. Monomer nach einem der Ansprüche 23 bis 25, wobei W eine dimerisierbare Gruppe ist, ausgewählt aus der Gruppe umfassend:
-C(O)-C(Z₂) = CH₂ 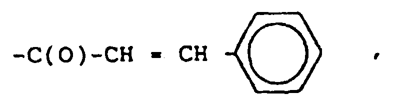 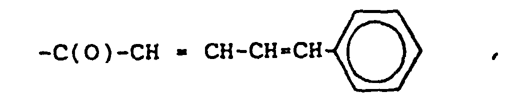 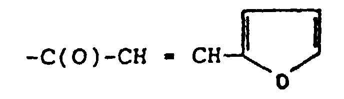
und 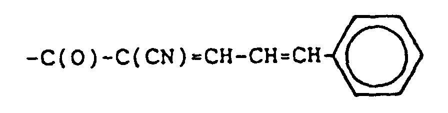 wobei Z₂ ein Wasserstoff-, Fluor- oder Chloratom oder eine Methylgruppe ist und die komplementäre funktionelle Ankergruppe ebenfalls eine dimerisierbare funktionelle Gruppe umfaßt, ausgewählt aus der gleichen Gruppe.

27. Monomer nach Anspruch 23 der nachfolgenden Formel (VII):

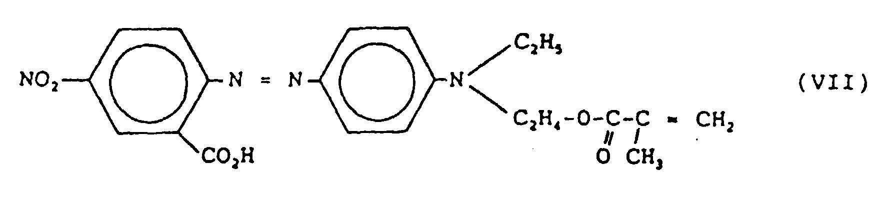

28. Verfahren zum Herstellen eines Monomers mit nicht-linearen optischen Eigenschaften nach Anspruch 23, wobei X = Y eine Azogruppe ist, aus den Verbindungen: 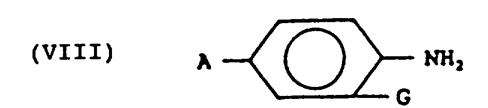 und 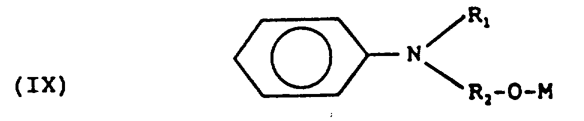 dadurch gekennzeichnet, daß es einen Diazotierungsschritt der Verbindung (VIII) umfaßt um ein Diazosalz dieser Verbindung zu erhalten, danach einen Kopplungsschritt des Diazosalzes und der Verbindung (IX).

29. Verfahren nach Anspruch 28, wobei M eine der Gruppe der Acryl- oder Methacrylreste angehörende Gruppe ist und wobei die Verbindung (IX) hergestellt wird durch Veresterung der Verbindung (X): 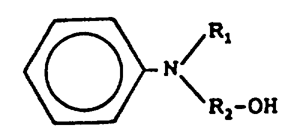 mit der Säure HO-M oder derem Säurechlorid.

30. Verfahren zur Herstellung eines Materials nach einem der Ansprüche 1 bis 21, wobei G eine -C(O)-O-(CH₂)ₙ-OH Gruppe ist und wobei die Gruppe G hergestellt wird durch Veresterung einer anfangs in dem Chromophor anstelle der Gruppe G vorhandenen -COOH Gruppe mit einer Verbindung Br-(CH₂)ₙ-OH.

31. Verfahren zur Herstellung eines Materials nach einem der Anspruche 1 bis 21, wobei G eine -C(O)-O-(CH₂)ₙ-O-W Gruppe ist und -O-W eine dimerisierbare Carboxylgruppe ist, umfassend einen Veresterungsschritt zwischen dem Alkohol
Br-(CH₂)ₙ-OH
und dem Säurechlorid Cl-W um die Verbindung (XI):
Br-(CH₂)ₙ-O-W
herzustellen, danach einen Veresterungsschritt zwischen der Verbindung (XI) und einer anfangs in dem Chromophor anstelle der Gruppe G vorhandenen -COOH Gruppe.
